(19) Europäisches Patentamt
European Patent Office
Office européen des brevets

(11) EP 3 521 909 A1

(12) # EUROPEAN PATENT APPLICATION
published in accordance with Art. 153(4) EPC

(43) Date of publication:
**07.08.2019 Bulletin 2019/32**

(21) Application number: **17856499.3**

(22) Date of filing: **29.09.2017**

(51) Int Cl.:
***G02C 7/10*** *(2006.01)* ***G02B 5/22*** *(2006.01)*
***C09K 3/00*** *(2006.01)*

(86) International application number:
**PCT/JP2017/035715**

(87) International publication number:
**WO 2018/062552 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2016 JP 2016195242**

(71) Applicant: **Hoya Lens Thailand Ltd.**
**Pathumthani 12130 (TH)**

(72) Inventors:
- **OHNISHI, Tomofumi**
  **Tokyo 160-8347 (JP)**
- **KOUSAKA, Masahisa**
  **Tokyo 160-8347 (JP)**
- **MIYAJIMA, Shinya**
  **Tokyo 160-8347 (JP)**

(74) Representative: **Beckmann, Claus et al**
**Kraus & Weisert**
**Patentanwälte PartGmbB**
**Thomas-Wimmer-Ring 15**
**80539 München (DE)**

(54) **METHOD FOR MANUFACTURING SPECTACLE LENS, AND SPECTACLE LENS**

(57) An object is to provide a method for manufacturing a spectacle lens having an excellent cut ratio of light having a wavelength of 420 nm and further having an excellent appearance, and a spectacle lens.
[1] A method for manufacturing a spectacle lens, including a step of immersing a substrate in an immersion liquid containing an indole-based UV absorber, and [2] a spectacle lens including a substrate impregnated with an indole-based UV absorber by a dyeing method.

EP 3 521 909 A1

## Description

### Technical Field

The present disclosure relates to a method for manufacturing a spectacle lens, and a spectacle lens.

### Background Art

In an optical member such as a spectacle lens, by cutting light rays in a blue region (wavelength region of 380 to 500 nm), glare is reduced, and visibility and contrast are improved. In addition, it is said that light rays in a blue region (380 to 500 nm) damage the retina or the like due to strong energy with respect to health of the eyes. Damage due to blue light is referred to as "blue light hazard". Particularly light around 420 nm on a low wavelength side is dangerous, and it is said that light in this region is desirably cut.

Patent Literature 1 describes a light shielding lens for protective eyeglasses that is obtained by blending a UV absorber with a synthetic resin spectacle lens and shields ultraviolet/visible light rays having a predetermined wavelength or less, characterized in that ultraviolet/visible light rays having a wavelength of 430 nm or less are shielded by blending 0.01 to 2 parts by mass of an indole-based UV absorber having a melting point of 140 to 150°C relative to 100 parts by mass of a synthetic resin.

### Citation List

### Patent Literature

Patent Literature 1: JP 2012-58643 A

### Summary of Invention

### Technical Problem

According to Patent Literature 1, it is possible to increase a cut ratio of light having a wavelength around 420 nm. However, when a spectacle lens is manufactured by blending an indole-based UV absorber with a synthetic resin raw material and then curing the resulting mixture as in the manufacturing method described in the Literature, the indole-based UV absorber is denatured, has a brown color tone, and has an appearance impaired disadvantageously. In particular, when the spectacle lens has a brownish color tone, even if the color tone is corrected by a bluing agent or the like, a favorable color tone cannot be obtained, and there is a problem from a viewpoint of optical characteristics.

An object of an Example of the present disclosure is to provide a method for manufacturing a spectacle lens having an excellent cut ratio of light having a wavelength of 420 nm and further having excellent optical characteristics, and a spectacle lens.

### Solution to Problem

The present inventors have found that a spectacle lens having an excellent cut ratio of light having a wavelength of 420 nm and further having excellent optical characteristics can be obtained by immersing a substrate in an immersion liquid containing an indole-based UV absorber for permeation.

An Example of the present disclosure relates to the following [1] and [2].

[1] A method for manufacturing a spectacle lens, including
a step of immersing a substrate in an immersion liquid containing an indole-based UV absorber.
[2] A spectacle lens including a substrate impregnated with an indole-based UV absorber by a dyeing method.

### Advantageous Effects of Invention

According to the above-described Example, it is possible to provide a method for manufacturing a spectacle lens having an excellent cut ratio of light having a wavelength of 420 nm and further having excellent optical characteristics, and a spectacle lens.

Description of Embodiments

[Method for manufacturing spectacle lens]

An Example of the present disclosure includes a step of immersing a substrate in an immersion liquid containing an indole-based UV absorber.

<Immersion liquid>

The immersion liquid contains an indole-based UV absorber, preferably contains a surfactant, a carrier, a solvent, and the like. The immersion liquid preferably contains an indole-based UV absorber and a solvent, more preferably contains an indole-based UV absorber, a surfactant, and a solvent, and still more preferably contains an indole-based UV absorber, a surfactant, a carrier, and a solvent.

[Indole-based UV absorber]

The term "indole-based UV absorber" means a compound having an indole ring structure.
By selecting the indole-based UV absorber, a spectacle lens having an excellent cut ratio of light having a wavelength of 420 nm can be obtained.
The indole-based UV absorber is preferably, from a viewpoint of improving a cut ratio of light having a wavelength of 420 nm, a compound represented by formula (I) :

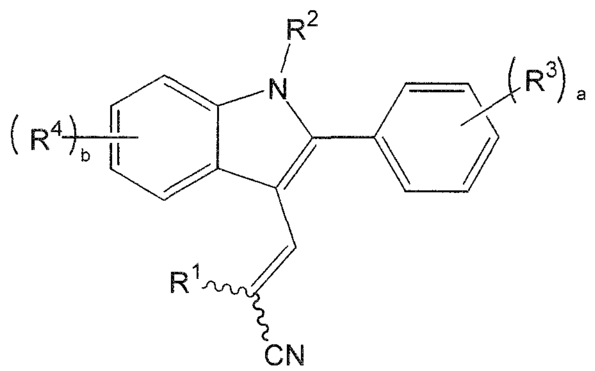

(I)

wherein in the formula, $R^1$ is $-COOR^{11}$ or -CN, $R^{11}$ is an alkyl group having 1 to 6 carbon atoms, $R^2$ is an alkyl group having 1 to 6 carbon atoms, $R^3$ and $R^4$ are each independently a halogen atom, an alkyl group having 1 to 6 carbon atoms, or an alkoxy group having 1 to 6 carbon atoms, and a and b are each independently an integer of 0 to 2.
$R^1$ is preferably $-COOR^{11}$ in which $R^{11}$ is an alkyl group having 1 to 3 carbon atoms. $R^1$ is more preferably-$COOCH_2CH_3$.
a and b are each preferably 0.
Examples of the indole-based UV absorber include (Z)-2-ethyl-2-cyano-3-(1-methyl-2-phenyl-1H-indol-3-yl) acrylate, (E)-2-ethyl-2-cyano-3-(1-methyl-2-phenyl-1H-indol-3-yl) acrylate (hereinafter also referred to simply as "2-ethyl-2-cyano-3-(1-methyl-2-phenyl-1H-indol-3-yl) acrylate" as an E-isomer or a Z-isomer), and 1-methyl-2-phenyl-3-(2,2-dicyanovinyl)-1H-indole.
These indole-based UV absorbers can be used singly or in combination of two or more kinds thereof.
The indole-based UV absorber is preferably ethyl-2-cyano-3-(1-methyl-2-phenyl-1H-indol-3-yl) acrylate or 1-methyl-2-phenyl-3-(2,2-dicyanovinyl)-1H-indole, and more preferably ethyl-2-cyano-3-(1-methyl-2-phenyl-1H-indol-3-yl) acrylate from a viewpoint of improving a cut ratio of light having a wavelength of 420 nm.
The content of the indole-based UV absorber in the immersion liquid is preferably 1 g/L or more, more preferably 5 g/L or more, and still more preferably 10 g/L or more. In addition, the content is preferably 40 g/L or less, more preferably 35 g/L or less, and still more preferably 30 g/L or less.

[Surfactant]

The immersion liquid preferably further contains a surfactant from a viewpoint of improving permeability of the indole-based UV absorber.
Examples of the surfactant include an anionic surfactant and a nonionic surfactant. Examples of the anionic

surfactant include alkylbenzene sulfonate, alkyl sulfosuccinate, and lauryl sulfate. Examples of the nonionic surfactant include a polyoxyethylene alkyl ether and a polyoxyethylene sorbitan fatty acid ester. These surfactants can be used singly or in combination of two or more kinds thereof.

Among these compounds, an anionic surfactant is preferable from a viewpoint of improving permeability of the indole-based UV absorber.

Examples of a commercially available surfactant include "Nicca Sunsolt 7000" (manufactured by Nicca Chemical Co., Ltd.) which is an anionic surfactant.

The content of the surfactant in an immersion liquid is preferably 1 mL/L or more, more preferably 10 mL/L or more, still more preferably 20 mL/L or more, and further still more preferably 30 mL/L or more. In addition, the content of the surfactant in the immersion liquid is preferably 100 mL/L or less, more preferably 80 mL/L or less, still more preferably 60 mL/L or less, and further still more preferably 50 mL/L or less.

[Career]

The carrier is a compound having an effect of promoting permeation of a UV absorber.

Examples of the carrier include an aromatic compound. Examples of the aromatic compound include an alcohol having an aromatic substituent, such as benzyl alcohol or cinnamyl alcohol, a phenol-based compound such as orthophenylphenol or paraphenylphenol, methylnaphthalene, various benzophenone-based compounds, monochlorobenzene, o-dichlorobenzene, m-dichlorobenzene, 1,2,4-trichlorobenzene, 1,2,5-trichlorobenzene, 1,3,5-trichlorobenzene, 1,2,3-trichlorobenzene, tetrachlorobenzene, pentachlorobenzene, hexachlorobenzene, monochloronaphthalene, and various polychloronaphthalene compounds. These carriers can be used singly or in combination of two or more kinds thereof.

Among these compounds, an alcohol having an aromatic substituent is preferable, benzyl alcohol and cinnamyl alcohol are more preferable, and benzyl alcohol is still more preferable from a viewpoint of improving permeability of the indole-based UV absorber.

The content of the carrier in the immersion liquid is preferably 1 mL/L or more, more preferably 2 mL/L or more, and still more preferably 3 mL/L or more. In addition, the content in the immersion liquid is preferably 30 mL/L or less, more preferably 20 mL/L or less, and still more preferably 10 mL/L or less.

The content of the carrier in the immersion liquid is preferably 1 part by mass or more, more preferably 5 parts by mass or more, still more preferably 10 parts by mass or more, further still more preferably 15 parts by mass or more, and further still more preferably 20 parts by mass or more relative to 100 parts by mass of the indole-based UV absorber. The content of the carrier is preferably 500 parts by mass or less, more preferably 200 parts by mass or less, still more preferably 100 parts by mass or less, further still more preferably 60 parts by mass or less, and further still more preferably 40 parts by mass or less relative to 100 parts by mass of the indole-based UV absorber.

[Solvent]

The solvent is preferably an aqueous solvent, and more preferably water.

The aqueous solvent is a solvent containing 60% by mass or more of water. The content of water in the aqueous solvent is preferably 70% by mass or more, more preferably 80% by mass or more, and still more preferably 90% by mass or more. In addition, the content of water in the aqueous solvent is 100% by mass or less, and more preferably 100% by mass.

Besides, the immersion liquid may contain a bluing agent or the like from a viewpoint of correcting a color tone.

<Substrate>

The substrate is, for example, a spectacle lens substrate.

Examples of a resin forming the substrate include a polycarbonate resin, a urethane urea resin, a (thio)urethane resin, an episulfide resin, a polyamide resin, and a polyester resin. The (thio)urethane resin means at least one selected from the group consisting of a thiourethane resin and a urethane resin. Among these resins, a polycarbonate resin, a urethane urea resin, a (thio)urethane resin, and an episulfide resin are preferable.

The substrate is preferably a substrate obtained by polymerizing a polymerizable composition containing a monomer, and more preferably a substrate obtained by polymerizing a polymerizable composition containing diethylene glycol bisallyl carbonate.

In order to obtain a three-dimensionally crosslinked optical resin, the monomer preferably contains a monomer having two or more polymerizable unsaturated bonds in a molecule thereof.

Examples of the polymerizable unsaturated bond include a (meth)acrylate group, an allyl group, and a vinyl group. Note that the (meth)acrylate group is at least one selected from the group consisting of a methacrylate group

and an acrylate group.

Among these groups, at least one selected from the group consisting of a methacrylate group and an allyl group is preferable.

The monomer having two or more polymerizable unsaturated bonds in a molecule thereof preferably contains diethylene glycol bisallyl carbonate, and more preferably contains diethylene glycol bisallyl carbonate, benzyl methacrylate, diallyl phthalate, and an alkyl methacrylate in which an alkyl group has 1 to 4 carbon atoms.

The blending amount of diethylene glycol bisallyl carbonate is preferably 5% by mass or more, more preferably 10% by mass or more, still more preferably 20% by mass or more, and preferably 100% by mass or less, more preferably 80% by mass or less, still more preferably 50% by mass or less, further still more preferably 40% by mass or less relative to the total amount of monomers.

In a case where diethylene glycol bisallyl carbonate is used in combination with benzyl methacrylate, diallyl phthalate, and an alkyl methacrylate in which an alkyl group has 1 to 4 carbon atoms, the blending amount of diethylene glycol bisallyl carbonate is more preferably 5% by mass or more, still more preferably 10% by mass or more, further still more preferably 20% by mass or more, and more preferably 40% by mass or less, still more preferably 35% by mass or less relative to the total amount of monomers.

The blending amount of benzyl methacrylate is preferably 5% by mass or more, more preferably 10% by mass or more, still more preferably 15% by mass or more, and preferably 40% by mass or less, more preferably 30% by mass or less, still more preferably 25% by mass or less relative to the total amount of monomers.

As diallyl phthalate, one or two selected from the group consisting of diallyl isophthalate and diallyl terephthalate can be mentioned.

The blending amount of diallyl phthalate is preferably 14% by mass or more, more preferably 20% by mass or more, still more preferably 30% by mass or more, and preferably 88% by mass or less, more preferably 70% by mass or less, still more preferably 60% by mass or less relative to the total amount of monomers.

As the alkyl methacrylate in which an alkyl group has 1 to 4 carbon atoms, at least one selected from the group consisting of methyl methacrylate, ethyl methacrylate, n-propyl methacrylate, iso-propyl methacrylate, n-butyl methacrylate, sec-butyl methacrylate, iso-butyl methacrylate, and tert-butyl methacrylate can be mentioned.

The blending amount of the alkyl methacrylate is preferably 1% by mass or more, more preferably 2% by mass or more, still more preferably 3% by mass or more, and preferably 6% by mass or less, more preferably 5% by mass or less relative to the total amount of monomers.

Examples of the radical initiator used in polymerization include 1,1-azobiscyclohexane carbonate, diisopropyl peroxycarbonate, 1,1'-azobiscyclohexane nitrate, and di-tert-butyl peroxide.

The blending amount of the radical initiator is preferably 0.1 parts by mass or more, more preferably 0.5 parts by mass or more, still more preferably 1.0 part by mass or more, and preferably 10 parts by mass or less, more preferably 8 parts by mass or less, and still more preferably 5 parts by mass or less relative to 100 parts by mass of monomers.

[Urethane urea resin]

The urethane urea resin contains, for example, a polymer of an isocyanate-terminated prepolymer which is a reaction product of an aliphatic diisocyanate having a cyclic structure in a molecule thereof and a diol having an average molecular weight of 300 to 2500 and an aromatic diamine. In addition, the urethane urea resin contains a phosphoric acid monoester or a phosphoric acid diester,

The aliphatic diisocyanate having a cyclic structure in a molecule thereof is an aliphatic diisocyanate having a cyclic structure in a main chain thereof or a side chain thereof. The cyclic structure may be an alicyclic ring, an aromatic ring, or a heterocyclic ring, but an alicyclic diisocyanate is preferable.

Examples of the alicyclic diisocyanate include 4,4'-methylenebis(cyclohexyl isocyanate), isophorone diisocyanate, 1,2-bis(isocyanatomethyl)cyclohexane, 1,3-bis(isocyanatomethyl)cyclohexane, 1,4-bis(isocyanatomethyl)cyclohexane, 1,2-diisocyanatocyclohexane, 1,3-diisocyanatocyclohexane, 1,4-diisocyanatocyclohexane, and norbornene diisocyanate.

Examples of the diisocyanate having an aromatic ring include m-xylylene diisocyanate, o-xylylene diisocyanate, p-xylylene diisocyanate, and m-tetramethyl xylylene diisocyanate. In particular, the diisocyanate having an aromatic ring is preferably at least one selected from the group consisting of 4,4'-methylenebis(cyclohexyl isocyanate), isophorone diisocyanate, 1,3-bis(isocyanatomethyl) cyclohexane, and norbornene diisocyanate.

The diol preferably has an average molecular weight of 400 to 1000.

Examples of the diol include polyoxyethylene glycol, polyoxypropylene glycol, polyoxytetramethylene glycol, a polyester diol formed of ethylene glycol and adipic acid, a polyester diol formed of propylene glycol and adipic acid, a polyester diol formed of diethylene glycol and adipic acid, a polyester diol formed of 1,4-butanediol and adipic acid, a polyester diol formed of neopentyl glycol and adipic acid, a polyester diol formed of 1,6-hexanediol and adipic acid, a polyester diol formed of 1,10-decanediol and adipic acid, a polyester diol formed of 1,4-butanediol and sebacic acid, a

polycaprolactone diol formed of ethylene glycol and ε-caprolactone, a polycaprolactone diol formed of propylene glycol and ε-caprolactone, a polycaprolactone diol formed of diethylene glycol and ε-caprolactone, a polycaprolactone diol formed of 1,4-butanediol and ε-caprolactone, a polycaprolactone diol formed of neopentyl glycol and ε-caprolactone, a polycaprolactone diol formed of 1,6-hexanediol and ε-caprolactone, a polycaprolactone diol formed of 1,10-decanediol and ε-caprolactone, and polycarbonate glycol. Among these compounds, polyoxypropylene glycol, polyoxytetramethylene glycol, a polyester diol formed of 1,4-butanediol and adipic acid, a polyester diol formed of neopentyl glycol and adipic acid, a polyester diol formed of 1,6-hexanediol and adipic acid, and a polyester diol formed of 1,10-decanediol and adipic acid are preferable.

Examples of the aromatic diamine include 1,3,5-trimethyl-2,4-diaminobenzene, 1,3,5-trimethyl-2,6-diaminobenzene, 1,3,5-triethyl-2,4-diaminobenzene, 1,3,5-triethyl-2,6-diaminobenzene, 1,3,5-trithiomethyl-2,4-diaminobenzene, 1,3,5-trithiomethyl-2,6-diaminobenzene, 3,5-diethyl-2,4-diaminotoluene, 3,5-diethyl-2,6-diaminotoluene, 3,5-dithiomethyl-2,4-diaminotoluene, 3,5-dithiomethyl-2,6-diaminotoluene, 1-ethyl-3,5-dimethyl-2,4-diaminobenzene, 1-ethyl-3,5-dimethyl-2,6-diaminobenzene, 1-ethyl-3,5-dithiomethyl-2,4-diaminobenzene, 1-ethyl-3,5-dithiomethyl-2,6-diaminobenzene, 1-thiomethyl-3,5-dimethyl-2,4-diaminotoluene, 1-thiomethyl-3,5-dimethyl-2,6-diaminotoluene, 1-thiomethyl-3,5-diethyl 2,4-diaminotoluene, 1-thiomethyl-3,5-diethyl-2,6-diaminotoluene, 3-ethyl-5-thiomethyl-2,4-diaminotoluene, 3-ethyl-5-thiomethyl-2,6-diaminotoluene, and 3-thiomethyl-5-ethyl-2,4-diaminotoluene. Among these compounds, 3,5-diethyl-2,4-diaminotoluene, 3,5-diethyl-2,6-diaminotoluene, 3,5-dithiomethyl-2,4-diaminotoluene, 3,5-dithiomethyl-2,6-diaminotoluene, and the like can be mentioned.

As a ratio between the isocyanate-terminated prepolymer and the aromatic diamine, a molar ratio of an isocyanate group of the prepolymer to an amino group of the aromatic diamine is preferably 1.00 to 1.15, and more preferably 1.02 to 1.12.

[(Thio)urethane resin]

Examples of the (thio)urethane resin include a polymer of a polyisocyanate compound and a polythiol compound and a polymer of a polyisocyanate compound and a polyol compound.

Examples of the polyisocyanate compound include an alicyclic isocyanate compound such as bis(isocyanatomethyl) cyclohexane, bis(isocyanatomethyl) bicyclo[2.2.1]heptane, hydrogenated 2,6-tolylene diisocyanate, hydrogenated meta- and para-phenylene diisocyanate, hydrogenated 2,4-tolylene diisocyanate, hydrogenated diphenylmethane diisocyanate, hydrogenated metaxylylene diisocyanate, hydrogenated paraxylylene diisocyanate, or isophorone diisocyanate; an isocyanate compound having no alicyclic ring or aromatic ring, such as meta- and para-phenylenediisocyanate, 2,6-tolylene diisocyanate, 2,4-tolylene diisocyanate, 4,4'-diphenylmethane diisocyanate, meta- and para-xylylene diisocyanate [bis(isocyanatomethyl) benzene], meta- and para-tetramethylxylylene diisocyanate, 2,6-naphthalene diisocyanate, 1,5-naphthalene diisocyanate, hexamethylene diisocyanate, octamethylene diisocyanate, 2,2,4-trimethylhexamethylene diisocyanate, tetramethylene diisocyanate, a biuret reaction product of hexamethylene diisocyanate, a trimer of hexamethylene diisocyanate, lysine diisocyanate, lysine triisocyanate, 1,6,11-undecane triisocyanate, or triphenylmethane triisocyanate; and a sulfur-containing isocyanate compound such as diphenyl disulfide-4,4'-diisocyanate, 2,2'-dimethyldiphenyl disulfide-5,5'-diisocyanate, 3,3'-dimethyldiphenyl disulfide-5,5'-diisocyanate, 3,3'-dimethyldiphenyl disulfide-6,6'-disocyanate, 4,4'-dimethyldiphenyl disulfide-5,5'-diisocyanate, 3,3'-dimethoxydiphenyl disulfide-4,4'-diisocyanate, 4,4'-dimethoxydiphenyl disulfide-3,3'-diisocyanate, diphenyl sulfone-4,4'-diisocyanate, diphenyl sulfone-3,3'-diisocyanate, benzylidene sulfone-4,4'-diisocyanate, diphenylmethanesulfone-4,4'-diisocyanate, 4-methyldiphenylmethanesulfone-2,4'-diisocyanate, 4,4'-dimethoxydiphenylsulfone-3,3'-diisocyanate, 3,3'-dimethoxy-4,4'-diisocyanatodibenzylsulfone, 4,4'-dimethyldiphenylsulfone-3,3'-diisocyanate, 4,4'-di-tert-butyldiphenylsulfone-3,3'-diisocyanate, 4,4'-dimethoxybenzene ethylene disulfone-3,3'-diisocyanate, 4,4'-dichlorodiphenylsulfone-3,3'-diisocyanate, 4-methyl-3-isocyanatobenzenesulfonyl-4'-isocyanatophenol ester, 4-methoxy-3-isocyanatobenzenesulfonyl-4'-isocyanatophenol ester, 4-methyl-3-isocyanatobenzenesulfonylanilide-3'-methyl-4'-isocyanate, dibenzenesulfonyl-ethylenediamine-4,4'-diisocyanate, 4,4'-dimethoxybenzenesulfonylethylenediamine-3,3'-diisocyanate, 4-methyl-3-isocyanatobenzenesulfonylanilide-4-methyl-3'-isocyanate, thiophene-2,5-diisocyanate, thiophene-2,5-diisocyanatomethyl, 1,4-dithiane-2,5-diisocyanate, 1,4-dithiane-2,5-diisocyanatomethyl, 1,4-dithiane-2,3-diisocyanatomethyl, 1,4-dithiane-2-isocyanatomethyl-5-isocyanatopropyl, 1,3-dithiolane-4,5-diisocyanate, 1,3-dithiolane-4,5-diisocyanatomethyl, 1,3-dithiolane-2-methyl-4,5-diisocyanatomethyl, 1,3-dithiolane-2,2-diisocyanatoethyl, tetrahydrothiophene-2,5-diisocyanate, tetrahydrothiophene-2,5-diisocyanatomethyl, tetrahydrothiophene-2,5-diisocyanatoethyl, or tetrahydrothiophene-3,4-diisocyanatomethyl. Among these compounds, an alicyclic isocyanate compound is preferable.

Examples of the polythiol compound include an aliphatic thiol such as methanedithiol, 1,2-ethanedithiol, 1,1-propanedithiol, 1,2-propanedithiol, 1,3-propanedithiol, 2,2-propanedithiol, 1,6-hexanedithiol, 1,2,3-propanetrithiol, tetrakis(mercaptomethyl) methane, 1,1-cyclohexanedithiol, 1,2-cyclohexanedithiol, 2,2-dimethylpropane-1,3-dithiol, 3,4-dimethoxybutane-1,2-dithiol, 2-methylcyclohexane-2,3-dithiol, 1,1-bis(mercaptomethyl) cyclohexane, thiomalic acid bis(2-mercaptoethylester), 2,3-dimercaptosuccinic acid (2-mercaptoethyl ester), 2,3-dimercapto-1-propanol (2-mercaptoacetate), 2,3-dimercapto-1-propanol (3-mercaptoacetate), diethylene glycol bis(2-mercaptoacetate), diethylene glycol bis(3-mercaptopropionate), 1,2-dimercaptopropyl methyl ether, 2,3-dimercaptopropyl methyl ether, 2,2-bis(mercaptomethyl)-1,3-propanediol, bis(2-mercaptoethyl) ether, ethylene glycol bis(2-mercaptoacetate), ethylene glycol bis(3-mercaptopropionate), trimethylolpropane tris(2-mercaptoacetate), trimethylolpropane tris(3-mercaptopropionate), pentaerythritol tetrakis(2-mercaptoacetate), pentaerythritol tetrakis(3-mercaptopropionate), or 1,2-bis(2-mercaptoethylthio)-3-mercaptopropane; an aromatic thiol such as 1,2-dimercaptobenzene, 1,3-dimercaptobenzene, 1,4-dimercaptobenzene, 1,2-bis(mercaptomethyl)benzene, 1,3-bis(mercaptomethyl) benzene, 1,4-bis(mercaptomethyl) benzene, 1,3-bis(mercaptoethyl)benzene, 1,4-bis(mercaptoethyl) benzene, 1,2-bis(mercaptomethoxy) benzene, 1,3-bis(mercaptomethoxy)benzene, 1,4-bis(mercaptomethoxy) benzene, 1,2-bis(mercaptoethoxy) benzene, 1,3-bis(mercaptoethoxy)benzene, 1,4-bis(mercaptoethoxy)benzene, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl) benzene, 1,3,5-tris(mercaptomethyl)benzene, 1,2,3-tris(mercaptoethyl)benzene, 1,2,4-tris(mercaptoethyl) benzene, 1,3,5-tris(mercaptoethyl)benzene, 1,2,3-tris(mercaptomethoxy)benzene, 1,2,4-tris(mercaptomethoxy) benzene, 1,3,5-tris(mercaptomethoxy) benzene, 1,2,3-tris(mercaptoethoxy)benzene, 1,2,4-tris(mercaptoethoxy) benzene, 1,3,5-tris(mercaptoethoxy)benzene, 1,2,3,4-tetramercaptobenzene, 1,2,3,5-tetramercaptobenzene, 1,2,4,5-tetramercaptobenzene, 1,2,3,4-tetrakis(mercaptomethyl)benzene, 1,2,3,5-tetrakis(mercaptomethyl)benzene, 1,2,4,5-tetrakis(mercaptomethyl)benzene, 1,2,3,4-tetrakis(mercaptoethyl)benzene, 1,2,3,5-tetrakis(mercaptoethyl)benzene, 1,2,4,5-tetrakis(mercaptoethyl)benzene, 1,2,3,4-tetrakis(mercaptoethyl)benzene, 1,2,3,5-tetrakis(mercaptomethoxy)benzene, 1,2,4,5-tetrakis(mercaptomethoxy)benzene, 1,2,3,4-tetrakis(mercaptoethoxy)benzene, 1,2,3,5-tetrakis(mercaptoethoxy)benzene, 1,2,4,5-tetrakis(mercaptoethoxy)benzene, 2,2'-dimercaptobiphenyl, 4,4'-dimercaptobiphenyl, 4,4'-dimercaptobibenzyl, 2,5-toluenedithiol, 3,4-toluenedithiol, 1,4-naphthalenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-di(p-methoxyphenyl)propane-2,2-dithiol, 1,3-diphenylpropane-2,2-dithiol, phenylmethane-1,1-dithiol, or 2,4-di(p-mercaptophenyl)pentane; a halogen-substituted aromatic thiol including a chlorine-substituted product and a bromine-substituted product, such as 2,5-dichlorobenzene-1,3-dithiol, 1,3-di(p-chlorophenyl)propane-2,2-dithiol, 3,4,5-tribromo-1,2-dimercaptobenzene, or 2,3,4,6-tetrachloro-1,5-bis(mercaptomethyl)benzene; an aromatic thiol containing a sulfur atom in addition to a mercapto group, such as 1,2-bis(mercaptomethylthio)benzene, 1,3-bis(mercaptomethylthio)benzene, 1,4-bis(mercaptoethylthio) benzene, 1,2-bis(mercaptoethylthio)benzene, 1,3-bis(mercaptoethylthio)benzene, 1,4-bis(mercaptoethylthio) benzene, 1,2,3-tris(mercaptomethylthio)benzene, 1,2,4-tris(mercaptomethylthio)benzene, 1,3,5-tris(mercaptomethylthio)benzene, 1,2,3-tris(mercaptoethylthio)benzene, 1,2,4-tris(mercaptoethylthio)benzene, 1,3,5-tris(mercaptoethylthio)benzene, 1,2,3,4-tetrakis(mercaptomethylthio)benzene, 1,2,3,5-tetrakis(mercaptomethylthio)benzene, 1,2,4,5-tetrakis(mercaptomethylthio)benzene, 1,2,3,4-tetrakis(mercaptoethylthio)benzene, 1,2,3,5-tetrakis(mercaptoethylthio)benzene, 1,2,4,5-tetrakis(mercaptoethylthio)benzene, or nucleus alkylated products thereof; an aliphatic thiol containing a sulfur atom in addition to a mercapto group, such as bis(mercaptomethyl)sulfide, bis(mercaptoethyl)sulfide, bis(mercaptopropyl)sulfide, bis(mercaptomethylthio) methane, bis(2-mercaptoethylthio)methane, bis (3-mercaptopropropylthio) methane, 1,2-bis(mercaptomethylthio) ethane, 1,2-bis(2-mercaptoethylthio)ethane, 1,2-bis(3-mercaptopropylthio)ethane, 1,3-bis(mercaptomethylthio) propane, 1,3-bis(2-mercaptoethylthio)propane, 1,3-bis(3-mercaptopropylthio)propane, 1,2-bis(2-mercaptoethylthio)-3-mercaptopropane, 2-mercaptoethylthio-1,3-propanedithiol, 1,2,3-tris(mercaptomethylthio)propane, 1,2,3-tris(2-mercaptoethylthio)propane, 1,2,3-tris(3-mercaptopropylthio)propane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl)sulfide, 2,5-dimercapto-1,4-dithiane, bis(mercaptomethyl)disulfide, bis(mercaptoethyl) disulfide, bis(mercaptopropyl)disulfide, thioglycolates thereof, mercaptopropionates thereof, hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercaptopropionate), hydroxypropyl sulfide bis(2-mercaptoacetate), hydroxypropyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), hydroxypropyl disulfide bis(2-mercaptoacetate), hydroxypropyl disulfide bis(3-mercaptopropionate), 2-mercaptoethyl ether bis(2-mercaptoacetate), 2-mercaptoethyl ether bis(3-mercaptopropionate), 1,4-dithiane-2,5-diol bis(2-mercaptoacetate), 1,4-dithiane-2,5-diol bis(3-mercaptopropionate), thioglycolic acid (2-mercaptoethyl ester), thiodipropionic acid bis(2-mercaptoethyl ester), 4,4'-thiodibutyric acid bis(2-mercaptoethyl ester), dithiodiglycolic acid bis(2-mercaptoethyl ester), dithiodipropionic acid bis(2-mercaptoethyl ester), 4,4'-dithiodibutyric acid bis(2-mercaptoethyl ester), thiodiglycolic acid bis(2,3-dimercaptopropyl ester), thiodipropionic acid bis(2,3-dimercaptopropyl ester), dithiodiglycolic acid bis(2,3-dimercaptopropyl ester), dithiodipropionic acid bis(2,3-dimercaptopropyl ester), 4-mercaptomethyl-3,6-dithiaoctane-1,8-dithiol, bis(mercaptomethyl)-3,6,9-trithia-1,11-undecanedithiol, or bis(1,3-dimercapto-2-propyl) sulfide; and a heterocyclic compound containing a sulfur atom in addition to a mercapto group, such as 3,4-thiophenedithiol, tetrahydrothiophene-2,5-dimercaptomethyl, 2,5-dimercapto-1,3,4-thiadiazole, 2,5-dimercapto-1,4-dithiane, or 2,5-dimercaptomethyl-1,4-dithiane.

Examples of the polyol compound include an aliphatic polyol such as ethylene glycol, diethylene glycol, propylene glycol, dipropylene glycol, butylene glycol, neopentyl glycol, glycerin, trimethylolethane, trimethylolpropane, butanetriol, 1,2-methyl glucoside, pentaerythritol, dipentaerythritol, tripentaerythritol, triethylene glycol, polyethylene glycol, tris(2-hydroxyethyl)isocyanurate, cyclobutanediol, cyclopentanediol, cyclohexanediol, cycloheptanediol, cyclooctanediol, bicyclo[4.3.0]-nonanediol, dicyclohexanediol, tricyclo[5.3.1.1]dodecanediol, spiro[3.4]octanediol, or butyl hexanediol; an aromatic polyol such as dihydroxy naphthalene, trihydroxy naphthalene, tetrahydroxynaphthalene, dihydroxy benzene, benzene triol, trihydroxy phenanthrene, bisphenol A, bisphenol F, xylylene glycol, or tetrabromobisphenol A, and an addition reaction product thereof with an alkylene oxide such as ethylene oxide or propylene oxide; bis-[4-(hydroxyethoxy)phenyl]sulfide, bis-[4-(2-hydroxypropoxy) phenyl]sulfide, bis-[4-(2,3-dihydroxypropoxy)phenyl] sulfide, bis-[4-(4-hydroxycyclohexyloxy)phenyl]sulfide, bis-[2-methyl-4-(hydroxyethoxy)-6-butylphenyl]sulfide, and a compound obtained by adding ethylene oxide and/or propylene oxide with an average of 3 molecules or less per hydroxy group to each of these compounds; and a polyol containing a sulfur atom, such as di-(2-hydroxyethyl) sulfide, 1,2-bis-(2-hydroxyethylmercapto)ethane, bis (2-hydroxyethyl) disulfide, 1,4-dithiane-2,5-diol, bis(2,3-dihydroxypropyl) sulfide, tetrakis(4-hydroxy-2-thiabutyl) methane, bis(4-hydroxyphenyl)sulfone (trade name: bisphenol S), tetrabromobisphenol S, tetramethylbisphenol S, 4,4'-thiobis(6-tert-butyl-3-methylphenol), or 1,3-bis(2-hydroxyethylthioethyl)-cyclohexane.

In addition, in order to modify physical properties such as heat resistance and refractive index, for example, in addition to a monomer forming an episulfide resin described below, another monomer such as diethylene glycol allyl carbonate can be added to these monomers.

In a case where a (thio)urethane resin is used as the substrate, the (thio)urethane resin is polymerized from a raw material in which the total mass of the polyisocyanate compound and the polythiol compound is preferably 60 parts by mass or more, more preferably 80 parts by mass or more, and still more preferably 90 parts by mass or more relative to 100 parts by mass of the total amount of monomers.

[Episulfide resin]

Examples of the episulfide resin include a polymer formed of a monomer containing a monomer having an episulfide group (epithio group). Examples of the monomer having an episulfide group include an episulfide compound having an alicyclic skeleton, such as 1,3- and 1,4-bis(β-epithiopropylthio) cyclohexane, 1,3- and 1,4-bis(β-epithiopropylthiomethyl) cyclohexane, bis[4-(β-epithiopropylthio)cyclohexyl]methane, 2,2-bis[4-(β-epithiopropylthio)cyclohexyl]propane, or bis[4-(β-epithiopropylthio)cyclohexyl]sulfide; an episulfide compound having an aromatic skeleton, such as 1,3- and 1,4-bis(β-epithiopropylthio)benzene, 1,3- and 1,4-bis(β-epithiopropylthiomethyl)benzene, bis[4-(β-epithiopropylthio)phenyl]methane, 2,2-bis[4-(β-epithiopropylthio)phenyl]propane, bis[4-(β-epithiopropylthio)phenyl]sulfide, bis[4-(β-epithiopropylthio)phenyl]sulfine, or 4,4-bis(β-epithiopropylthio)biphenyl; an episulfide compound having a dithiane ring skeleton, such as 2,5-bis(β-epithiopropylthiomethyl)-1,4-dithiane, 2,5-bis(β-epithiopropylthioethyl thiomethyl)-1,4-dithiane, 2,5-bis(β-epithiopropylthioethyl)-1,4-dithiane, or 2,3,5-tri(β-epithiopropylthioethyl)-1,4-dithiane; and an episulfide compound having an aliphatic skeleton, such as 2-(2-β-epithiopropylthioethylthio)-1,3-bis(β-epithiopropylthio) propane, 1,2-bis[(2-β-epithiopropylthioethyl)thio]-3-(β-epithiopropylthio)propane, tetrakis(β-epithiopropylthiomethyl)methane, 1,1,1-tris(β-epithiopropylthiomethyl)propane, or bis-(β-epithiopropyl) sulfide.

In order to modify lens physical properties such as impact resistance and processability, for example, it is also possible to add another monomer for an optical member, such as the above-described monomers for forming a (thio)urethane resin.

In addition, a diethylene glycol bisallyl carbonate-based monomer can be added to the monomer forming a (thio)urethane resin or an episulfide resin.

As the diethylene glycol bisallyl carbonate-based monomer, diethylene glycol bisallyl carbonate alone and a monomer mixture of diethylene glycol bisallyl carbonate and a monomer copolymerizable with diethylene glycol bisallyl carbonate are applicable. Specific examples of the copolymerizable monomer include an aromatic vinyl compound such as styrene, α-methylstyrene, vinyltoluene, chlorostyrene, chloromethylstyrene, or divinylbenzene; a mono (meth)acrylate such as methyl (meth)acrylate, n-butyl (meth)acrylate, n-hexyl (meth)acrylate, cyclohexyl (meth)acrylate, 2-ethylhexyl (meth)acrylate, methoxydiethylene glycol (meth)acrylate, methoxypolyethylene glycol (meth)acrylate, 3-chloro-2-hydroxypropyl (meth)acrylate, stearyl (meth)acrylate, lauryl (meth)acrylate, phenyl (meth)acrylate, glycidyl (meth)acrylate, or benzyl methacrylate; a mono (meth)acrylate having a hydroxy group, such as 2-hydroxyethyl (meth)acrylate, 2-hydroxypropyl (meth)acrylate, 3-hydroxypropyl (meth)acrylate, 3-phenoxy-2-hydroxypropyl (meth)acrylate, or 4-hydroxybutyl (meth)acrylate; a di(meth)acrylate such as ethylene glycol di(meth)acrylate, diethylene glycol di(meth)acrylate, triethylene glycol di(meth)acrylate, polyethylene glycol di(meth)acrylate, 1,3-butylene glycol di(meth)acrylate, 1,6-hexanediol di(meth)acrylate, neopentyl glycol di(meth)acrylate, polypropylene glycol di(meth)acrylate, 2-hydroxy-1,3-di(meth)acryloxypropane, 2,2-bis[4-(meth)acryloxyethoxy)phenyl]propane, 2,2-bis[4-((meth)acryloxy-diethoxy)phenyl]propane, or 2,2-bis[4-((meth)acryloxy-polyethoxy)phenyl]propane; a tri(meth)acrylate such as trimethylolpropane trimethacrylate or tetramethylolmethane trimethacrylate; a tetra(meth)acrylate such as tetramethylolmethane tetra(meth)acrylate; diallyl phthalate, diallyl isophthalate, and diallyl terephthalate.

In a case where the episulfide resin is used as the substrate, the amount of a monomer having an episulfide group is preferably 60 parts by mass or more, more preferably 80 parts by mass or more, and still more preferably 90 parts by mass or more relative to 100 parts by mass of the total amount of monomers.
The thickness and diameter of the substrate are not particularly limited. However, the thickness is usually about 1 to 30 mm, and the diameter is usually about 50 to 100 mm.
The substrate has a refractive index ne of preferably 1.53 or more, more preferably 1.55 or more, still more preferably 1.58 or more, further still more preferably 1.60 or more, further still more preferably 1.67 or more, and preferably 1.80 or less, more preferably 1.70 or less.

(Pretreatment of substrate)

The substrate may be subjected to a pretreatment such as a cleaning treatment or a surface treatment before immersion from a viewpoint of enhancing affinity with the indole-based UV absorber.
Examples of the cleaning treatment include an ozone treatment and a plasma treatment. If a surface of the substrate to be dyed is subjected to the ozone treatment or the plasma treatment, an organic substance attached to the surface of the substrate is removed, and hydrophilicity of the surface of the substrate is enhanced. Therefore, affinity between an immersion liquid and the surface of the substrate is considered to be improved.
The ozone treatment and the plasma treatment are not particularly limited, and it is only required to perform a cleaning treatment using a known ozone treatment apparatus or plasma treatment apparatus. A plasma output in the plasma treatment is preferably 50 to 500 W, more preferably 100 to 300 W, and still more preferably 200 to 300 W. The degree of vacuum is preferably substantially vacuum pressure (for example, the degree of vacuum is $1 \times 10^{-3}$ to $1 \times 10^{4}$ Pa, more preferably $1 \times 10^{-3}$ to $1 \times 10^{3}$ Pa, and still more preferably $1 \times 10^{-2}$ to $5 \times 10^{2}$ Pa).

<Immersion conditions>

The temperature of the immersion liquid when the substrate is immersed therein is preferably 50°C or higher, more preferably 60°C or higher, and still more preferably 70°C or higher. The temperature is preferably 95°C or lower, more preferably 98°C or lower, and still more preferably 95°C or lower.
Immersion time in the immersion liquid is preferably one minute or more, and more preferably two minutes or more. In addition, the immersion time is preferably 120 minutes or less, and more preferably 60 minutes or less.
The substrate withdrawn from the immersion liquid may be subjected to water washing or a drying treatment if necessary. Conditions such as drying temperature and drying time can be appropriately selected.
In the obtained substrate, the cut ratio of light having a wavelength of 420 nm is preferably 40% or more, more preferably 50% or more, still more preferably 60% or more, further still more preferably 65% or more, and further still more preferably 70% or more. In addition, the cut ratio of the light having a wavelength of 420 nm is preferably 90% or less, more preferably 85% or less, and still more preferably 80% or less. The cut ratio of the light having a wavelength of 420 nm is a value obtained from a transmittance of the light having a wavelength of 420 nm obtained by measuring a transmission spectrum using an ultraviolet-visible spectrophotometer.
The obtained substrate has a luminous transmittance of preferably 70% or more, more preferably 75% or more, still more preferably 80% or more in order to secure transparency of the spectacle lens. The luminous transmittance is preferably 99% or less, more preferably 98% or less, and still more preferably 95% or less. The luminous transmittance is a value obtained by the method prescribed in JIS T7333-2005.
The obtained substrate has a YI value of preferably 18 or less, more preferably 15 or less, still more preferably 13 or less in order to secure transparency of the spectacle lens. The YI value is preferably 3 or more, more preferably 5 or more, and still more preferably 8 or more.
The YI value (yellowness index) is a value obtained by a method specified in JIS K7103-1977.
The method for manufacturing a spectacle lens according to an embodiment of the present disclosure may include a step of further laminating a functional layer on the substrate after the immersion step.
As the functional layer, for example, at least one selected from the group consisting of a hard coat layer, a primer layer, an antireflection film, and a water repellent film can be mentioned.
The hard coat layer is disposed for improving scratch resistance and preferably can be formed by applying a coating liquid containing an organic silicon compound, a fine particulate inorganic substance such as tin oxide, silicon oxide, zirconium oxide, or titanium oxide, or the like.
The primer layer is disposed for improving impact resistance, and contains, for example, polyurethane as a main component. Here, the content of polyurethane is preferably 50% by mass or more in the primer layer.
Examples of the antireflection film include a film obtained by laminating silicon oxide, titanium dioxide, zirconium oxide, tantalum oxide, or the like.
The water repellent film can be formed using an organic silicon compound having a fluorine atom.

[Spectacle lens]

The spectacle lens is a spectacle lens obtained by the above-described method, that is, a spectacle lens in which a substrate is impregnated with an indole-based UV absorber by a dyeing method.

In the spectacle lens, the cut ratio of light having a wavelength of 420 nm is preferably 40% or more, more preferably 50% or more, still more preferably 60% or more, further still more preferably 65% or more, and further still more preferably 70% or more. In addition, the cut ratio of the light having a wavelength of 420 nm is preferably 90% or less, more preferably 85% or less, and still more preferably 80% or less.

The spectacle lens has a luminous transmittance of preferably 70% or more, more preferably 80% or more, still more preferably 90% or more in order to secure transparency of the spectacle lens. The luminous transmittance is preferably 99% or less, more preferably 98% or less, and still more preferably 95% or less.

The spectacle lens has a YI value of preferably 18 or less, more preferably 15 or less, still more preferably 13 or less in order to secure transparency of the spectacle lens. The YI value is preferably 3 or more, more preferably 5 or more, and still more preferably 8 or more.

In an embodiment according to the present disclosure, as for the examples of components, contents, and physical properties, matters exemplified or described as a preferable range in the detailed description of the invention may be combined with each other arbitrarily.

In addition, by adjusting the composition described in Examples so as to be the composition described in the detailed description of the invention, an embodiment according to the present disclosure can be performed in a similar manner to Examples in the entire claimed composition range.

[Examples]

Hereinafter, specific Examples will be described, but the present claims are not limited by the following Examples.

[Optical characteristics]

An appearance of a lens is observed under a three-wavelength fluorescent lamp, and color tone adjustment is possible by bluing for a yellow color tone judged according to the following criteria. Meanwhile, color tone correction is impossible by bluing for a brown color tone, and it is judged that optical characteristics are not suitable.

A: Light yellow color tone
B: Dark yellow color tone
C: Brown color tone

[Cut ratio of light having wavelength of 420 nm]

A transmission spectrum was measured using an ultraviolet-visible spectrophotometer "U-4100" (manufactured by Hitachi, Ltd.), and a cut ratio was calculated from the transmittance of light having a wavelength of 420 nm using the following formula.

$$420\text{ nm cut ratio} = [1 - \text{transmittance of light having wavelength of 420 nm}] \times 100$$

[YI value]

A YI value was measured according to the plastic yellowness and yellowing degree test method specified in JIS K7103-1977.

Manufacture Example 1: Immersion liquid 1

20 g/L of an indole-based UV absorber (compound name: (Z)-2-ethyl-2-cyano-3-(1-methyl-2-phenyl-1H-indol-3-yl) acrylate, CAS No. 102311-49-9), 40 mL/L of a surfactant "NIKKA SUNSOLT 7000" (manufactured by Nicca Chemical Co., Ltd.), and benzyl alcohol (manufactured by Tokyo Chemical Industry Co., Ltd.) as a carrier were mixed with water so as to obtain a concentration of 5 mL/L, and immersion liquid 1 was prepared.

Manufacture Examples 2 to 4: Immersion liquids 2 to 4

Immersion liquids 2 to 4 were prepared in a similar manner to Example 1 except that the immersion liquid was changed so as to have the compositions illustrated in Table 1.

[Table 1]

| Manufacture Example | | 1 | 2 | 3 | 4 |
|---|---|---|---|---|---|
| Immersion liquid | | 1 | 2 | 3 | 4 |
| UV absorber | Indole-based UV absorber 1 | 20 g/L | 20 g/L | | |
| | Indole-based UV absorber 2 | | | 20 g/L | |
| | Benzophenone-based UV absorber 1 | | | | 20 g/L |
| Surfactant | Nicca Sunsolt | 40 mL/L | 40 mL/L | 40 mL/L | 40 mL/L |
| Carrier | Benzyl alcohol | 5 mL/L | | 5 mL/L | 5 mL/L |
| | Cinnamyl alcohol | | 5 g/L | | |
| Water | | Residue | Residue | Residue | Residue |

Indole-based UV absorber 1: (Z)-2-ethyl-2-cyano-3-(1-methyl-2-phenyl-1H-indol-3-yl) acrylate
Indole-based UV absorber 2: 1-methyl-2-phenyl-3-(2,2-dicyanovinyl)-1H-indole
Benzophenone-based UV absorber 1: 2,2'-dihydroxy-4-methoxybenzophenone
Nicca Sunsolt: "Nicca Sunsolt 7000" (manufactured by Nikka Chemical Co., Ltd.)

Example 1

While the immersion liquid 1 was kept at a liquid temperature of 95°C, a spectacle lens substrate "HILUX 1.5" (HOYA Corporation) made of diethylene glycol bisallyl carbonate and having a refractive index of 1.50 was immersed therein for one hour for permeation. Various evaluations were performed and results thereof are illustrated in Table 2.

Examples 2 to 6 and Comparative Example 3

The spectacle lenses were prepared in a similar manner to Example 1 except that the immersion liquids and the substrates of the spectacle lenses were as illustrated in Table 2. Various evaluations were performed and results thereof are illustrated in Table 2.

Comparative Example 1

Relative to 100 parts by mass of a mixture obtained by adding diisopropyl peroxydicarbonate as a polymerization initiator to a liquid monomer of an ADC resin CR-39 (diethylene glycol bisallyl carbonate), 1.5 parts by mass of an indole-based UV absorber (compound name: (Z)-ethyl-2-cyano-3-(1-methyl-2-phenyl-1H-indol-3-yl) acrylate, CAS No. 102311-49-9)) was blended. The resulting mixture was mixed and stirred together with an appropriate amount of a solvent and vacuum-degassed to prepare a liquid molding material. This liquid molding material was injected into a molding die obtained by attaching a gasket to two glass molds and having a thickness of 2.2 mm. This molding die was put into an electric furnace, gradually heated from 15°C to 100°C over 20 hours, and held for two hours. After completion of polymerization, the molding die was taken out from the electric furnace, and the resulting product was released from the die to obtain a spectacle lens. The obtained spectacle lens was further annealed at 100°C for three hours. Various evaluations were performed and results thereof are illustrated in Table 2.

Comparative Example 2

A spectacle lens was manufactured in a similar manner to Comparative Example 2 except that the amount of the indole-based UV absorber was changed from 1.5 parts by mass to 0.3 parts by mass. Various evaluations were performed and results thereof are illustrated in Table 2.

[Table 2]

| | Immersion liquid | | Substrate | Optical characteristics | Cut ratio of light of 420 nm | YI value |
|---|---|---|---|---|---|---|
| | No. | UV absorber | | | | |
| Example 1 | 1 | Indole-based UV absorber 1 | 1.50 | A | 75 | 11.9 |
| Example 2 | 2 | Indole-based UV absorber 1 | 1.50 | A | 50 | 5.3 |
| Example 3 | 3 | Indole-based UV absorber 2 | 1.50 | A | 70 | 9.8 |
| Example 4 | 1 | Indole-based UV absorber 1 | 1.53 | A | 73 | 11.1 |
| Example 5 | 1 | Indole-based UV absorber 1 | 1.60 | A | 66 | 9.2 |
| Example 6 | 1 | Indole-based UV absorber 1 | 1.67 | A | 67 | 9.3 |
| Comparative Example 1 | - *1 | | - | C | 100 | 35 |
| Comparative Example 2 | - *1 | | - | C | 32 | 20 |
| Comparative Example 3 | 4 | Benzophenone-based UV absorber 1 | 1.50 | A | 5 | 0.8 |
| *1: Added before polymerization of substrate raw material without using immersion liquid | | | | | | |

1.50: "HILUX 1.5" (allyl carbonate resin manufactured by HOYA Corporation)
1.53: "Phoenix" (urethane urea resin manufactured by HOYA Corporation)
1.60: "EYAS" (thiourethane resin manufactured by HOYA Corporation)
1.67: "EYNOA" (thiourethane resin manufactured by HOYA Corporation)

According to Examples 1 to 6, as compared with the case where the indole-based UV absorber is blended before polymerization of a resin in Comparative Examples 1 and 2, it is found that it is possible to suppress discoloration and to obtain a favorable appearance by immersing the indole-based UV absorber in the substrate by an immersion method. By comparing Example 1 with Comparative Example 3, it is found that excellent results are obtained at the cut ratio of light having a wavelength of 420 nm by using the indole-based UV absorber. By comparing Example 1 with Example 2, it is found that better results are obtained at the cut ratio of light having a wavelength of 420 nm by using benzyl alcohol as a carrier. In addition, from the results of Examples 1 and 4 to 6, it is found that the indole-based UV absorber can be immersed in a substrate by the immersion method in a wide range of substrates.

Finally, an embodiment of the present disclosure will be summarized.

An embodiment of the present disclosure relates to a method for manufacturing a spectacle lens, including a step of immersing a substrate in an immersion liquid containing an indole-based UV absorber.

According to the above-described embodiment, it is possible to provide a method for manufacturing a spectacle lens having an excellent cut ratio of light having a wavelength of 420 nm and further having excellent optical characteristics, and a spectacle lens.

The embodiment disclosed herein is exemplary in all respects, and it should be considered that the embodiment is not restrictive. The scope of the present disclosure is defined not by the above description but by claims, and intends to include all modifications within meaning and a scope equal to claims.

## Claims

1. A method for manufacturing a spectacle lens, comprising
a step of immersing a substrate in an immersion liquid containing an indole-based UV absorber.

2. The method for manufacturing a spectacle lens according to claim 1, wherein a cut ratio of light of 420 nm of the substrate after immersion is 40% or more.

3. The method for manufacturing a spectacle lens according to claim 1 or 2, wherein
the indole-based UV absorber is a compound represented by formula (I):

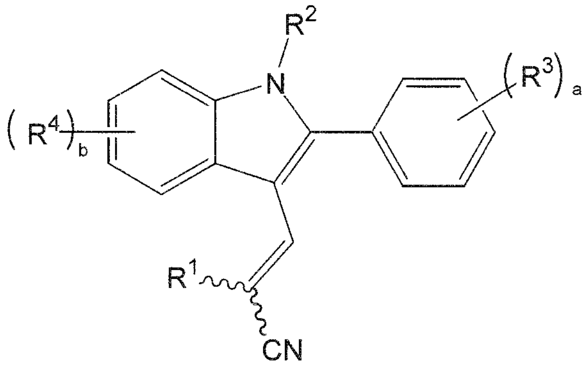

(I)

wherein, in the formula, $R^1$ is $-COOR^{11}$ or -CN, $R^{11}$ is an alkyl group having 1 to 6 carbon atoms, $R^2$ is an alkyl group having 1 to 6 carbon atoms, $R^3$ and $R^4$ are each independently a halogen atom, an alkyl group having 1 to 6 carbon atoms, or an alkoxy group having 1 to 6 carbon atoms, and a and b are each independently an integer of 0 to 2.

4. The method for manufacturing a spectacle lens according to any one of claims 1 to 3, wherein the indole-based UV absorber is at least one selected from the group consisting of 2-ethyl-2-cyano-3-(1-methyl-2-phenyl-1H-indol-3-yl) acrylate and 1-methyl-2-phenyl-3-(2,2-dicyanovinyl)-1H-indole.

5. The method for manufacturing a spectacle lens according to any one of claims 1 to 4, wherein the substrate contains at least one resin selected from the group consisting of a polycarbonate resin, a urethane urea resin, a (thio)urethane resin, and an episulfide resin.

6. The method for manufacturing a spectacle lens according to any one of claims 1 to 5, wherein the substrate is a substrate obtained by polymerizing a polymerizable composition containing diethylene glycol bisallyl carbonate.

7. The method for manufacturing a spectacle lens according to any one of claims 1 to 6, wherein the immersion liquid further contains a surfactant.

8. The method for manufacturing a spectacle lens according to any one of claims 1 to 7, wherein the immersion liquid further contains an alcohol having an aromatic substituent.

9. The method for manufacturing a spectacle lens according to claim 8, wherein the alcohol having an aromatic substituent is benzyl alcohol.

10. The method for manufacturing a spectacle lens according to any one of claims 1 to 9, wherein the substrate after immersion has a luminous transmittance of 70% or more.

11. A spectacle lens comprising a substrate,
wherein the substrate is impregnated with an indole-based UV absorber by a dyeing method.

**INTERNATIONAL SEARCH REPORT**

International application No. PCT/JP2017/035715

A. CLASSIFICATION OF SUBJECT MATTER
*G02C7/10*(2006.01)i, *G02B5/22*(2006.01)i, *C09K3/00*(2006.01)n

According to International Patent Classification (IPC) or to both national classification and IPC

B. FIELDS SEARCHED

Minimum documentation searched (classification system followed by classification symbols)
G02C7/00-7/16, G02B3/00-3/14, G02B5/20-5/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2013-54275 A (Nikon-Essilor Co., Ltd.), 21 March 2013 (21.03.2013), claims; paragraphs [0001], [0004] to [0010], [0017], [0024], [0029], [0043] to [0047]; fig. 2 (Family: none) | 1-6,10-11<br>7-9 |
| Y | JP 2001-159747 A (Hoya Corp.), 12 June 2001 (12.06.2001), paragraph [0017] (Family: none) | 7-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 October 2017 (20.10.17) | 07 November 2017 (07.11.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No. PCT/JP2017/035715

C (Continuation). DOCUMENTS CONSIDERED TO BE RELEVANT

| Category* | Citation of document, with indication, where appropriate, of the relevant passages | Relevant to claim No. |
|---|---|---|
| A | JP 2012-58643 A (Talex Optical Co., Ltd.), 22 March 2012 (22.03.2012), claims; paragraphs [0011] to [0021] & US 2013/0194658 A1 claims; paragraphs [0013] to [0025] & EP 2618206 A1 & AU 2011303181 A & CN 103261949 A | 1-11 |
| A | JP 4-134065 A (Orient Chemical Industries, Ltd.), 07 May 1992 (07.05.1992), claims; page 2, upper right column, line 1 to lower left column, line 10 & US 5296519 A claims; column 1, line 54 to column 2, line 22 & US 5401438 A & EP 477844 A1 & DE 69123915 T | 1-11 |
| A | JP 1-230003 A (Seiko Epson Corp.), 13 September 1989 (13.09.1989), claims; page 1, lower right column, line 20 to page 2, upper right column, line 4 (Family: none) | 1-11 |
| A | WO 2004/068215 A1 (Menicon Co., Ltd.), 12 August 2004 (12.08.2004), claims; page 11, lines 11 to 23; page 16, line 8 to page 19, line 7 & US 2005/0243273 A1 claims; paragraphs [0034], [0046] to [0050] & EP 1589367 A1 | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012058643 A